(19) Europäisches Patentamt
European Patent Office
Office européen des brevets

(11) Publication number: **0 000 564**
**B1**

(12) # EUROPEAN PATENT SPECIFICATION

(45) Date of publication of patent specification: **19.05.82**

(21) Application number: **78100469.2**

(22) Date of filing: **21.07.78**

(51) Int. Cl.³: **B 01 J 23/88,**
B 01 J 27/18, B 01 J 27/02,
C 07 C 121/32,
C 07 C 120/14

(54) **Catalysts containing iron and molybdenum and the use of these catalysts in the preparation of acrylonitrile or methacrylonitrile.**

(30) Priority: **28.07.77 US 819732**

(43) Date of publication of application:
**07.02.79 Bulletin 79/3**

(45) Publication of the grant of the patent:
**19.05.82 Bulletin 82/20**

(84) Designated Contracting States:
**BE CH DE FR GB NL SE**

(56) References cited:
**FR - A - 2 138 601**
**FR - A - 2 166 099**
**FR - A - 2 227 257**
**FR - A - 2 228 538**
**FR - A - 2 231 669**
**FR - A - 2 232 524**
**FR - A - 2 233 315**
**FR - A - 2 247 438**
**FR - A - 2 279 706**
**FR - A - 2 302 993**
**FR - A - 2 308 609**
**FR - A - 2 333 770**

(73) Proprietor: **THE STANDARD OIL COMPANY**
**Midland Building**
**Cleveland, Ohio 44115 (US)**

(72) Inventor: **Grasselli, Robert Karl**
**150 Greentree Road**
**Chagrin Falls Ohio 44022 (US)**
Inventor: **Suresh, Dev Dhanaraj**
**1052 Iroquois Run**
**Macedonia Ohio 44056 (US)**
Inventor: **Friedrich, Maria Strada**
**11211 Buckingham Avenue**
**Cleveland Ohio 44120 (US)**
Inventor: **Orndoff, David Allan**
**5519 Warner Hollow Road**
**Windsor Ohio 44099 (US)**

(74) Representative: **Redies, Bernd, Dr. rer. nat. et al,**
**Redies, Redies, Türk & Gille, Patentanwälte**
**Brucknerstrasse 20**
**D-4000 Düsseldorf 13 (DE)**

(56) References cited:
**FR - A - 2 354 812**
**FR - A - 2 357 512**
**NL - A - 74 08765**
**NL - A - 75 08637**
**US - A - 4 065 507**

**EP 0 000 564 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Catalysts containing iron and molybdenum and the use of these catalysts in the preparation of acrylonitrile or methacrylonitrile

## Technical field of the invention

The invention is related to new catalysts containing iron, bismuth, molybdenum and various additional elements which exhibit extremely attractive catalytic properties for the oxidation and ammoxidation of various olefins. The invention is further related to the use of such catalysts in the ammoxidation of propylene and isobutylene to acrylonitrile and, respectively, methacrylonitrile.

## Background of the invention

Catalysts for the oxidation, ammoxidation and oxydehydrogenation of olefins are well known. See, for example, U.S. 3,642,930 and U.S. 3,414,631, the disclosures of which are incorporated herein by reference. Further note commonly assigned US-applications Serial No. 490,532 filed July 22, 1974, Serial No. 380,527 filed July 19, 1973, and Serial No. 717,838 filed August 26, 1976, the disclosures of which are also incorporated herein by reference. Although these catalysts of the art are very desirable, the catalysts of the present invention have significant advantages over these other catalysts.

## Summary and detailed description of the invention

The present invention is based on the discovery that certain oxidation catalysts based on iron, bismuth and molybdenum and containing additional specified promoter elements exhibit excellent catalytic activity for a variety of olefin oxidation, ammoxidation, dehydrogenation and oxydehydrogenation reactions. In particular, it has been found that specific catalytic compositions as described below are especially suited for use in producing acrylonitrile and methacrylonitrile by the ammoxidation of propylene and isobutylene, respectively. In addition to being excellent ammoxidation catalysts for the production of acrylonitrile or methacrylonitrile, these catalysts also exhibit excellent catalytic activity for the conversion of olefins having four or more contiguous carbon atoms to the corresponding diolefins, and in the oxydehydrogenation of olefins to diolefins. The present invention however is particularly directed to a process for producing acrylonitrile or methacrylonitrile by the reaction of propylene or isobutylene, molecular oxygen and ammonia at a temperature of about 200°C to 600°C in the presence of the novel catalysts of the invention hereinafter defined.

In accordance with the present invention, novel catalysts having improved catalytic properties are represented by the following empirical formula

$$J_aQ_bZ_cFe_dD_eMo_{12}O_x$$

wherein

J is an alkali metal, thallium, silver or mixtures thereof;

Q is Co, Ni, Zn, Cd, Be, Mg, Ca, Sr, Ba, Ra or mixtures thereof;

Z is a two-or-more element system comprising Cu+W, Cu+Sn, Ce+W, Pr+Mn, Mn+W, W+Sb, Cr+Sn, W+Sn, Ge+Sn, Sb+Sn, Cr+Cu, Sb+Cu, or mixtures thereof;

D is Bi or Te; and

wherein

a is a number from 0.01 to 0.5;
b is a number from 0.1 to 20;
c is a number from 0.1 to 9;
d is a number from 0.1 to 20;
e is a number from 0.1 to 20; and

x is a value such that the valence requirement of the elements in the catalysts for oxygen are satisfied;

each element in said two or more element system being present in an amount of at least 1 atom percent based on the atoms in the system.

In this catalyst composition, the Z component is composed of systems of two or more specified elements. In these systems as described above, each element of the system is present in an amount of at least 1 atom percent, preferably at least 5 atom percent, based on the atoms in the particular system selected. In this catalyst, D is preferably Bi.

Preferably, the catalysts of this invention contains the above-indicated elements in the following amounts:

a is 0.03 to 0.5,
b is 4 to 10,
c is 0.5 to 7,
d is 0.5 to 5, and
e is 0.5 to 5.

In a particularly preferred embodiment, the foregoing preferred and optimal catalysts contain K, Rb and/or Cs and optionally Ni and/or Co.

Also, it is desirable if Z is selected so that the ratio of the amount of the first element of each system as described above to the amount of second element in the system is 1:0.25 to 1:0.75.

Of the foregoing catalysts, catalyst in which the Z component is selected from the following combinations of elements are of special interest: Cu+W, Cu+Sn, Ce+W, Pr+Mn, W+Sb, Cr+Sn, W+Sn, Ge+Sn, Sb+Sn, Cr+Cu, Sb+Cu or Mn+W; while those catalysts in which Z is one of Cu+W, Cu+Sn, Ce+W and Pr+Mn are particularly noteworthy.

The catalysts of this invention can be prepared in any conventional manner known in the art, such as for example the various techniques shown in the patents and applications referred to in the background of the invention. For example, the catalysts can be made by forming an aqueous composition of decomposable salts and/or oxides of the metals to be incorporated into the catalyst, evaporating the water from the aqueous composition to form a thick paste, drying the thick paste and then calcining the thick paste at elevated temperature in an oxidizing atmosphere. Techniques for forming catalysts of the type described herein are well known in the art, and those skilled in the art should have no difficulty in making catalysts of the invention.

The catalysts of the present invention may be used unsupported or supported on various conventional carriers such as $SiO_2$, $Al_2O_3$, $ZrO_2$, $TiO_2$, $BPO_4$, $SbPO_4$, MgO, montmorillonite, or the like. They may be used in fixed-bed or fluid-bed reactors as desired. When using these catalysts, they are simply substituted for the catalysts previously employed, and the reaction is conducted under substantially the same conditions.

For example, when the inventive catalysts are used in the ammoxidation of propylene or isobutylene to form acrylonitrile or methacrylonitrile, respectively, the olefin to be reacted together with oxygen and ammonia in conventional proportions are contacted with the catalyst at an elevated temperature, usually about 200—600°C in a fixed or fluid-bed reactor for a time sufficient to effect the appropriate ammoxidation reaction. Similarly, when the inventive catalysts are employed to catalyze other well known reactions, the reaction conditions employed are those which are conventional.

Specific embodiments

Comparative example A

$$80\%\ K_{0.1}Ni_{2.5}Co_{4.5}Fe_3BiP_{0.5}Mo_{12}O_x+20\%\ SiO_2$$

A solution of 127.1 g ammonium heptamolybdate $(NH_4)_6Mo_7O_{24}\cdot 4H_2O$ and water was prepared. To this solution was added 6.9 g of a 42.5% solution of $H_3PO_4$ and 102.7 g of 40% silica sol to form a slurry. Separately, an aqueous solution containing 72.7 g ferric nitrate, $Fe(NO_3)_3\cdot 9H_2O$; 29.1 g bismuth nitrate, $Bi(NO_3)_3\cdot 5H_2O$: 78.6 g cobalt nitrate $Co(NO_3)_2\cdot 6H_2O$; 43.6 g nickel nitrate $Ni(NO_3)_2\cdot 6H_2O$: and 6.1 g of a 10% potassium nitrate solution was prepared. The solution of metal nitrates was slowly added to the slurry. The resulting slurry was evaporated to dryness, and the solid obtained was heat treated at 290°C for three hours, at 425°C for three hours and at 550°C for 16 hours.

Examples 1 to 3

In the same manner as discussed in Comparative Example A, catalysts listed in the following Table I were prepared. Each of these catalysts as well as Comparative Catalyst A was tested for its catalytic activity in the ammoxidation of propylene to acrylonitrile. In carrying out this test, each of the catalysts was charged into a 5 ccm fixed-bed reactor. A feed of propylene/ammonia/oxygen/nitrogen/steam in amounts of 1.8/2.2/3.6/2.4/6 was fed to the reactor such that the WWH was 0.1 g propylene per gram catalyst per hour and the contact time was three seconds. The reaction temperature was maintained at 420°C. The results obtained as well as the results obtained when Comparative Catalyst A was employed was set forth in the following Table I.

Table I

| Example | Catalyst composition (80% Catalyst supported on 20% $SiO_2$) | Acrylonitrile per pass conversion | Acrylonitrile selectivity |
|---|---|---|---|
| Comp. A | $K_{0.1}Ni_{2.5}Co_{4.5}Fe_3BiP_{0.5}Mo_{12}O_x$ | 73.1 | 79.4 |
| 1 | $MnW_{0.5}(K_{0.1}Ni_{2.5}Co_{4.5}Fe_2BiMo_{12}O_x)$ | 68.5 | 86.4 |
| 2 | $SnMn_{0.5}(K_{0.1}Ni_{2.5}Co_{4.5}Fe_2BiMo_{12}O_x)$ | 76.2 | 82.8 |
| 3 | $PrMn_{0.5}(K_{0.1}Ni_{2.5}Co_{4.5}Fe_2BiMo_{12}O_x)$ | 77.9 | 78.8 |

The parentheses used in Table I as well as the following Table II have no significance other than to clarify the difference between the various catalysts.

Examples 4 to 6

Using the various catalysts prepared in the manner described above, additional examples in which

propylene was ammoxidized to acrylonitrile were conducted. These additional experiments were conducted in accordance with the same procedure used in Examples 1 to 3, except that the reaction temperature was 430°C, the contact time was 6 seconds and the catalyst support was 50% $SiO_2$. The results obtained are set forth in the following Table II.

Table II

| Example | Catalyst composition (80% Catalyst supported on 20% $SiO_2$) | Acrylonitrile per pass conversion | Acrylonitrile selectivity |
|---|---|---|---|
| Comp. A | $K_{0.1}Ni_{2.5}Co_{4.5}Fe_3BiP_{0.5}Mo_{12}O_x$ | 76.4 | 79.2 |
| Comp. A | $K_{0.1}Ni_{2.5}Co_{4.5}Fe_3BiP_{0.5}Mo_{12}O_x$ | 76.0 | 77.8* |
| 4 | $SnMn_{0.5}K_{0.2}(Ni_{2.5}Co_{4.5}Fe_2Mo_{12}O_x)$ | 77.0 | 77.4 |
| 5 | $CeW_{0.5}K_{0.3}(Ni_{2.5}Co_{4.5}Fe_2Mo_{12}O_x)$ | 80.0 | 81.1 |
| 6 | $GeSb_{0.5}K_{0.3}(Ni_{2.5}Co_{4.5}Fe_2Mo_{12}O_x)$ | 80.9 | 82.0* |

*9 second contact time

## Claims

1. Oxidation catalysts characterized by the general formula:

$$J_aQ_bZ_cFe_dD_eMo_{12}O_x$$

wherein

J is an alkali metal, thallium, silver or mixtures thereof;

Q is Co, Ni, Zn, Cd, Be, Mg, Ca, Sr, Ba, Ra or mixtures thereof;

Z is a two-or-more element system comprising Cu+W, Cu+Sn, Ce+W, Pr+Mn, Mn+W, W+Sb, Cr+Sn, W+Sn, Ge+Sn, Sb+Sn, Cr+Cu, Sb+Cu, or mixtures thereof;

D is Bi or Te; and

wherein

a is a number from 0.01 to 0.5;

b is a number from 0.1 to 20;

c is a number from 0.1 to 9;

d is a number from 0.1 to 20;

e is a number from 0.1 to 20; and

x is a value such that the valence requirement of the elements in the catalyst for oxygen are satisfied;

each element in said two or more element system being present in an amount of at least 1 atom percent based on the atoms in the system.

2. The catalysts of claim 1 characterized in that D is Bi.

3. The catalysts of claim 2 characterized in that Z is either Cu+W, Cu+Sn, Ce+W, Pr+Mn, W+Sb, Cr+Sn, W+Sn, Ge+Sn, Sb+Sn, Cr+Cu, Sb+Cu or Mn+W.

4. The catalysts of claim 2 characterized in that said catalysts contain at least one of K, Rb, and Cs and at least one of Co and Ni.

5. Process for the preparation of acrylonitrile or methacrylonitrile by the reaction of propylene or isobutylene, molecular oxygen and ammonia at a temperature of about 200°C to 600°C in the presence of a catalyst, characterized in that a catalysts is used characterized by the general formula

$$J_aQ_bZ_cFe_dD_eMo_{12}O_x$$

wherein

J is an alkali metal, thallium, silver or mixtures thereof;

Q is Co, Ni, Zn, Cd, Be, Mg, Ca, Sr, Ba, Ra or mixtures thereof;

Z is a two-or-more element system comprising Ge+Sb, Cu+W, Cu+Sn, Ce+W, Pr+Mn, Sn+Mn, Mn+W, W+Sb, Cr+Sn, W+Sn, Ge+Sn, Sb+Sn, Sb+P, Cr+Cu, Sb+Cu, Mn+P or mixtures thereof with the proviso that said catalyst is free of Tl when Z is Mn+P;

D is Bi or Te; and

wherein

a is a number from 0.01 to 0.5;

b is a number from 0.1 to 20;

c is a number from 0.1 to 9;

d is a number from 0.1 to 20;

e is a number from 0.1 to 20; and

x is a value such that the valence requirement of the elements in the catalyst for oxygen are satisfied;

each element in said two or more element system being present in an amount of at least 1 atom percent based on the atoms in the system.

6. Process as claimed in claim 5 wherein D is Bi.

7. Process as claimed in claim 6 wherein J is at least one of K, Rb and Cs and Q is at least one of Co and Ni.

**Patentansprüche**

1. Oxydationskatalysator, gekennzeichnet durch die allgemeine Formel

$$J_aQ_bZ_cFe_dD_eMo_{12}O_x$$

worin

J ein Alkalimetall, Thallium, Silber oder Gemisch hiervon ist;

Q Co, Ni, Zn, Cd, Be, Mg, Ca, Sr, Ba, Ra ist oder Gemische hiervon;

Z ein Elementensystem aus zwei oder mehr Elementen ist, die die Verbindungspaare Cu+W, Cu+Sn, Ce+W, Pr+Mn, Mn+W, W+Sb, Cr+Sn, W+Sn, Ge+Sn, Sb+Sn, Cr+Cu, Sb+Cu oder Gemische hiervon umfaßt;

D Bi oder Te ist und

worin

a eine Zahl von 0,01 bis 0,5,

b eine Zahl von 0,1 bis 20,

c eine Zahl von 0,1 bis 9,

d eine Zahl von 0,1 bis 20,

e eine Zahl von 0,1 bis 20 ist und

x eine Zahl ist, die zur Absättigung der Valenzen der Elemente in dem Katalysator durch Sauerstoff entspricht;

wobei jedes der Element in dem vorstehend genannten System aus zwei oder mehr Elementen in einer Menge von mindestens 1 Atom-% auf der Grundlage der Atome im System anwesend ist.

2. Der Katalysator gemäß Anspruch 1, dadurch gekennzeichnet, daß D Bi ist.

3. Der Katalysator gemäß Anspruch 2, dadurch gekennzeichnet, daß Z entweder Cu+W, Cu+Sn, Ce+W, Pr+Mn, W+Sb, Cr+Sn, W+Sn, Ge+Sn, Sb+Sn, Cr+Cu, Sb+Cu oder Mn+W ist.

4. Der Katalysator gemäß Anspruch 2, dadurch gekennzeichnet, daß der Katalysator mindestens eines der Elemente K, Rb und Cs und mindestens eines der Elemente Co und Ni enthält.

5. Verfahren zur Herstellung von Acrylnitril oder Methacrylnitril durch Umsetzung von Propylen oder Isobutylen, molekularem Sauerstoff und Ammoniak bei einer Temperatur von etwa 200°C bis 600°C in Anwesenheit eines Katalysators, dadurch gekennzeichnet, daß ein Katalysator verwendet wird, der durch die allgemeine Formel

$$J_aQ_bZ_cFe_dD_eMo_{12}O_x$$

gekennzeichnet ist,

worin

J ein Alkalimetall, Thallium, Silber oder Gemisch hiervon ist;

Q Co, Ni, Zn, Cd, Be, Mg, Ca, Sr, Ba, Ra ist oder Gemische hiervon;

Z ein Elementensystem aus zwei oder mehr Elementen ist, die die Verbindungspaare Ge+Sb, Cu+W, Cu+Sn, Ce+W, Pr+Mn, Sn+Mn, Mn+W, W+Sb, Cr+Sn, W+Sn, Ge+Sn, Sb+Sn, Sb+P, Cr+Cu, Sb+Cu, Mn+P oder Gemische hiervon mit der Maßgabe, daß der Katalysator frei von Tl ist, wenn Z Mn+P ist;

D Bi oder Te ist und

worin

a eine Zahl von 0,01 bis 0,5,

b eine Zahl von 0,1 bis 20,

c eine Zahl von 0,1 bis 9,

d eine Zahl von 0,1 bis 20,

e eine Zahl von 0,1 bis 20 ist und

x eine Zahl ist, die zur Absättigung der Valenzen der Elemente in dem Katalysator durch Sauerstoff entspricht;

wobei jedes der Elemente in dem vorstehend genannten System aus zwei oder mehr Elementen in einer Menge von mindestens 1 Atom-% auf der Grundlage der Atome im System anwesend ist.

6. Verfahren gemäß Anspruch 5, dadurch gekennzeichnet, daß D Bi ist.

7. Verfahren gemäß Anspruch 6, dadurch gekennzeichnet, daß J mindestens eines der Elemente K, Rb und Cs und Q mindestens eines der Elemente Co und Ni ist.

**Revendications**

1. Catalysateurs d'oxidation caracterisés par la formule générale ci-après:

$$J_aQ_bZ_cFe_dD_eMo_{12}O_x$$

dans laquelle
J est un métal alcalin, du thallium, de l'argent ou leurs mélanges;
Q est du Co, Ni, Zn, Cd, Be, Mg, Ca, Sr, Ba, Ra ou leurs mélanges;
Z est un système a deux ou plusieurs éléments comprenant du Cu+W, Cu+Sn, Ce+W, Pr+Mn, Mn+W, W+Sb, Cr+Sn, W+Sn,Ge+Sn, Sb+Sn, Cr+Cu, Sb+Cu ou leurs mélanges;
D représente du Bi ou Te; et

dans laquelle
a est un nombre de 0,01 à 0,5;
b est un nombre de 0,1 à 20;
c est un nombre de 0,1 à 9;
d est un nombre de 0,1 à 20;
e est un nombre de 0,1 à 20; et
x représente une valeur satisfaisant les exigences de valence des éléments compris dans le catalysateur pour l'oxygène;
chacun des éléments du système à deux ou plusieur éléments étant présent en une quantité d'au moins 1 pourcent atomique par rapport au nombre d'atomes compris dans le système.

2. Les catalysateurs de la revendication 1, caractérisés en ce que D représente du Bi.

3. Les catalysateurs de la revendication 2, caractérisés en ce que Z représente ou bien du Cu+W, Cu+Sn, Ce+W, Pr+Mn, W+Sb, Cr+Sn, W+Sn, Ge+Sn, Sb+Sn, Cr+Cu, Sb+Cu, ou du Mn+W.

4. Les catalysateurs de la revendication 2, caractérisés en ce que ces catalysateurs comprennent au moins un des éléments K, Rb et Cs et au moins un des éléments Co et Ni.

5. Procédé pour la préparation d'acrylonitril ou du methacrylonitril par réaction de propylène ou d'isobutylène, d'oxygène moléculaire et d'ammonium à une température variant de 200°C à 600°C et en présence d'un catalysateur, caractérisé en ce qu'il est utilisé un catalysateur de la formule générale suivante

$$J_aQ_bZ_cFe_dD_eMo_{12}O_x$$

dans laquelle
J est un métal alcalin, du thallium, de l'argent ou leurs mélanges;
Q est du Co, Ni, Zn, Cd, Be, Mg, Ca, Sr, Ba, Ra ou leurs mélanges;
Z est un système à deux ou plusieurs éléments comprenant du Ge+Sb, Cu+W, Cu+Sn, Ce+W, Pr+Mn, Sn+Mn, Mn+W, W+Sb, Cr+Sn, W+Sn, Ge+Sn, Sb+Sn, Sb+P, Cr+Cu, Sb+Cu, Mn+P ou leurs mélanges, il étant entendu que le dit catalysateur ne contiendra pas de Tl lorsque Z représente du Mn+P;
D est du Bi ou du Te; et

dans laquelle
a est un nombre de 0,01 à 0,5;
b est un nombre de 0,1 à 20;
c est un nombre de 0,1 à 9;
d est un nombre de 0,1 à 20;
e est un nombre de 0,1 à 20; et
x représente une valeurs satisfaisant les exigences de valence des éléments compris dans le catalysateur pour l'oxygène;
chacun des éléments du système à deux ou plusieurs éléments étant présent en une quantité d'au moins 1 pourcent atomique par rapport au nombre d'atomes compris dans le système.

6. Procédé suivant la revendication 5 dans lequel D représente du Bi.

7. Procédé suivant la revendication 6 dans lequel J représente au moins un des éléments K, Rb et Cs et Q représente au moins un des éléments Co et Ni.